# EUROPEAN PATENT SPECIFICATION

(11) **EP 4 005 482 B1**
(45) Date of publication and mention of the grant of the patent: **24.06.2026**
(21) Application number: 21183521.0
(22) Date of filing: 02.07.2021
(51) Int. Cl.: A61B 5/00, A61B 5/369, A61B 5/372, G06N 3/044, G06N 3/09, G06N 3/0464, G16H 50/70, G16H 50/20, G16H 40/63, G06N 3/045, G06N 3/082, G06N 5/01, G06N 20/10, G16H 20/30, G06N 20/20, A61N 1/372, G06N 3/08, G16H 20/40, A61N 1/36, G16H 40/60, G06N 3/048

(54) **COMPUTER PROGRAM FOR TRAINING A NEUROLOGICAL CONDITION DETECTION ALGORITHM, METHOD OF PROGRAMMING AN IMPLANTABLE NEUROSTIMULATION DEVICE AND IMPLANTABLE NEUROSTIMULATION DEVICE**
COMPUTERPROGRAMM ZUM TRAINIEREN EINES ALGORITHMUS ZUR ERKENNUNG EINES NEUROLOGISCHEN ZUSTANDS, VERFAHREN ZUR PROGRAMMIERUNG EINER IMPLANTIERBAREN NEUROSTIMULATIONSVORRICHTUNG UND IMPLANTIERBARE NEUROSTIMULATIONSVORRICHTUNG
PROGRAMME INFORMATIQUE POUR L'APPRENTISSAGE D'UN ALGORITHME DE DÉTECTION D'ÉTAT NEUROLOGIQUE, PROCÉDÉ DE PROGRAMMATION D'UN DISPOSITIF DE NEUROSTIMULATION IMPLANTABLE ET DISPOSITIF DE NEUROSTIMULATION IMPLANTABLE

(30) Priority: 25.11.2020 EP 20209813
(43) Date of publication of application: 01.06.2022
(73) Proprietor: Precisis GmbH, 69117 Heidelberg (DE)
(72) Inventor: Manzouri, Farrokh, 79117 Freiburg im Breisgau (DE); Schulze-Bonhage. Andreas, 79104 Freiburg im Breisgau (DE); Dümpelmann, Matthias, 79106 Freiburg im Breisgau (DE)
(74) Representative: Kröncke, Rolf

(56) References cited:
- US-A1- 2020 337 645
- MANZOURI FARROKH ET AL: "An Optimized EEG-Based Seizure Detection Algorithm for Implantable Devices", 2021 10TH INTERNATIONAL IEEE/EMBS CONFERENCE ON NEURAL ENGINEERING (NER), IEEE, 4 May 2021 (2021-05-04), pages 995 - 998, XP033920178, DOI: 10.1109/NER49283.2021.9441348
- BIRJANDTALAB JAVAD ET AL: "Automated seizure detection using limited-channel EEG and non-linear dimension reduction", COMPUTERS IN BIOLOGY AND MEDICINE, NEW YORK, NY, US, vol. 82, 25 January 2017 (2017-01-25), pages 49 - 58, XP029929494, ISSN: 0010-4825, DOI: 10.1016/J.COMPBIOMED.2017.01.011
- TRUONG NHAN DUY ET AL: "Supervised learning in automatic channel selection for epileptic seizure detection", EXPERT SYSTEMS WITH APPLICATIONS, ELSEVIER, AMSTERDAM, NL, vol. 86, 29 May 2017 (2017-05-29), pages 199 - 207, XP085114586, ISSN: 0957-4174, DOI: 10.1016/J.ESWA.2017.05.055

## Description

The invention is related to a computer program for training a neurological condition detection algorithm to be used for seizure detection in an implantable neurostimulation device having a target electrode arrangement. This computer program is also referred to as the training computer program. The invention is further related to a method of programming an implantable neurostimulation device using such training computer program and to a computer program in the form of a neurological condition detection algorithm or classifier for detecting neurological conditions from EEG data which has been trained and/or is being trained by such training computer program. This computer program is also referred to as the neurological condition detection computer program. The invention is further related to a neurostimulation device running such a neurological condition detection computer program. The disclosure is further related to a method for treatment of a neurological condition using a neurostimulation device running such a neurological condition detection computer program.

Despite progress in the development of medication, a significant share of epilepsy patients are resistant to treatment with antiepileptic drugs. As only a minority of these patients are surgical candidates, there is a need for innovative therapeutic approaches. An alternative treatment concept for these patients is the application of electrical stimulation in the early phases of a seizure to interrupt its spread across the brain. This can be accomplished using a closed-loop system, where the electrical brain activity is recorded by a set of electrodes and continuously or intervallic monitored with a seizure detector which triggers electrical stimulation to the seizure onset zone (SOZ) via the same or different electrodes.

BIRJANDTALAB JAVAD ET AL propose an "Automated seizure detection using limited-channel EEG and non-linear dimension reduction", COMPUTERS IN BIOLOGY AND MEDICINE, NEW YORK, NY, US, vol. 82, 25 January 2017 (2017-01-25), pages 49-58, XP029929494, ISSN: 0010-4825. US 2020/0337645 A1 discloses a system and method for classifying and modulating brain behavioral states.

Therefore, there is a need for reliable and energy-efficient seizure detectors. It is an object of the invention to provide solutions for this need.

The invention is defined in the appended set of claims.

The neurological condition which is identified by the neurological activity maybe a seizure, a stroke, neuropathic pain, dementia, Parkinson, tinnitus, aphasia, or any other specific neurological event. In the following, the example of a seizure is mainly used for describing the disclosure. However, the disclosure shall also cover other types of neurological events.

The invention allows for an optimum adaption of the neurological condition detection algorithm to the "real" electrode configuration, namely the pattern of the target electrode arrangement. In this way, the neurological condition detection algorithm can be optimally adapted to the patient's individual neurological condition onset pattern and the individual EEG recording sites of the implanted electrodes where optimum detection of neurological events can be expected. The target electrode arrangement may comprise solely recording electrodes which are used in the neurostimulation device for recording EEG signals from the patient. It is also possible that the target electrode arrangement may comprise solely stimulation electrodes which are used in the neurostimulation device for outputting stimulation signals to the patient. Another possibility is that the target electrode arrangement may comprise a combination of recording electrodes and stimulation electrodes. In some cases, one, more or all of the electrodes of the target electrode arrangement may be used for both purposes, recording EEG signals and outputting stimulation signals, such that they are combined recording and stimulation electrodes.

Typically, the steps of the computer program are executed by a computer. However, the disclosure is also related to a method comprising the above mentioned steps and/or the following steps. The target electrode arrangement must be defined before the training computer program is run. For example, characteristic data of the target electrode arrangement can be input or programmed in the training computer program. The characteristic data of the target electrode may comprise geometrical data of the location, size and/or configuration of the electrodes of the target electrode arrangement. Generally, the EEG data used for the training comprise a number of measurements over time per recording electrode channel and per patient. The training computer program may need some further manual input, depending on the details which are included in the EEG data set input in the computer program. For example, the EEG data can already be tagged or labelled with information identifying the time and/or location of a neurological condition like seizure activity. Also, information identifying the time and/or location of the neurological condition maybe input manually. In the training step the neurological condition detection algorithm performs detection of neurological activity in the EEG data, and in the course of the training the results of the detection by the neurological condition detection algorithm are compared with the tagging information. Based upon the results of the comparison parameters of the neurological condition detection algorithm are optimized recursively, until a sufficient detection level of the neurological condition detection algorithm is achieved.

According to an embodiment of the disclosure, such electrode channels are selected out of the available electrode channels which are in closest proximity to the location of the identified neurological activity which corresponds to the neurological condition. This allows for an optimum spatial correlation of the expected neurological activity of the patient and the location of the electrodes of the target electrode arrangement.

According to an embodiment of the disclosure, such electrode channels are selected out of the available electrode channels which have the closest geometrical match with the electrodes of the target electrode arrangement. In this way, neurological condition detection using the electrodes of the target electrode arrangement can be optimized to a level which performs comparable to neurological condition detection using a relatively complex 10-20 or 10-10 EEG electrode system, without needing such high number of electrodes in the target electrode arrangement.

According to the invention, the electrodes of the target electrode arrangement are arranged in a pseudo-Laplacian pattern. This allows for highly localized EEG recording. Through the pseudo-Laplacian configuration, external signal noise and/or artifacts from muscle activity or movement will be reduced significantly, improving neurological condition detection.

According to an embodiment of the disclosure, such electrode channels are selected out of the available electrode channels which are arranged in a pseudo-Laplacian pattern. This allows for a close match of the selected electrode channels with the pattern of the target electrode arrangement in pseudo-Laplacian pattern. In a pseudo-Laplacian pattern, the selected electrode channels comprise a center electrode and at least two circumferential electrodes which surround the center electrode.

According to an embodiment of the invention, step d) comprises the steps:
d1) calculating linear combinations of the EEG data of the selected subset of electrode channels, e.g. calculating linear combinations representing bipolar or quadrupolar electrode channels,
d2) training the neurological condition detection algorithm by using calculated linear combinations of the EEG data.

In this way, further virtual electrode channels can be generated by such calculation steps. This allows for a refinement of the spatial signal resolution of the electrode pattern, without needing further hardware electrode channels.

According to an embodiment of the invention, exactly 5 electrode channels are selected out of the available electrode channels. This allows for an optimum match of the selected electrode channels with the target electrode arrangement having also five electrodes.

According to an embodiment of the invention, the computer program comprises at least two training cycles of the neurological condition detection algorithm:
a) in a first training cycle a general training of the neurological condition detection algorithm is done using the EEG data of one or more patients,
b) in a second training cycle a patient specific training of the neurological condition detection algorithm is done using the EEG data only of the patient to which the neurological condition detection algorithm shall be applied and/or using the EEG data of another patient having similar neurological condition onset pattern as the patient to which the neurological condition detection algorithm shall be applied.

In this way the training of neurological condition detection algorithm can be further optimized and accelerated.

According to an embodiment of the invention, the computer program is arranged for evaluating data tags which are assigned to the EEG data which are input in the computer which executes the computer program, wherein the data tags are used for selecting the subset of electrode channels out of the available electrode channels. The data tags may comprise information about the location of an electrode channel on the patients head during clinical measurements of the EEG signals.

Another embodiment of the invention is a method of programming an implantable neurostimulation device, comprising the following steps:
c) running a training computer program of the aforementioned kind on a computer,
d) programming the neurological condition detection algorithm trained by the computer program into the implantable neurostimulation device.

In this way, the same advantages as mentioned before can be achieved. In addition, for training purposes a different computer can be used than the computer of the implantable neurostimulation device. This allows for using a more powerful computer for running the training computer program, which saves significant time.

According to an embodiment of the invention, the implantable neurostimulation device is a closed-loop neurostimulator which is arranged for recording EEG signals, for calculating stimulation signals based upon the recorded EEG signals and for outputting the stimulation signals. In this way, the neurostimulation therapy can be optimized and specifically adapted to the needs of the individual patient. The stimulation signals are sent out via the electrodes of the target electrode arrangement. For recording the EEG signals the same electrodes of the target electrode arrangement can be used. It is also possible that different electrodes are used for recording the EEG signals and outputting the stimulation signals, for example electrodes which are specifically optimized for EEG signal recording and electrodes which are specifically optimized for signal outputting.

According to an embodiment of the invention, the electrodes of the target electrode arrangement are arranged in a pseudo-Laplacian configuration. This allows for optimum stimulation success when the electrodes are used for neurostimulation. Through the pseudo-Laplacian pattern deep stimulation can be provided to the patient even with an electrode pattern which is flat. According to the disclosure, the electrodes can comprise a center electrode which is surrounded by at least two stimulation electrodes. It is also possible that there is a higher number of stimulation electrodes surrounding the center electrode. According to the invention, four stimulation electrodes surround a center electrode.

According to an embodiment of the invention, in step g) the computer program is run on an external computer which is not part of the implantable neurostimulation device. Another embodiment which is described is a computer program in the form of a neurological condition detection algorithm or classifier for detecting neurological conditions from EEG data which has been trained and/or is being trained by a training computer program of the aforementioned kind. The computer program can be configured for implementation on a microcontroller or on a digital signal processor (DSP) or on a field programmable gate array (FPGA) or on an application specific integrated circuit (ASIC). According to an embodiment of the disclosure, the computer program is optimized for lowest power consumption. According to another embodiment of the disclosure, the computer program is optimized for highest performance. According to another embodiment of the disclosure, the computer program is optimized for lowest power consumption under defined performance constraints. According to another embodiment of the disclosure, the computer program is optimized for highest performance under defined power constraints.

According to an embodiment of the disclosure, the neurological condition detection algorithm or classifier for detecting neurological conditions is an artificial intelligence algorithm, e.g. Random Forest, Support Vector Machine, Multi-layer Perceptron, Convolutional Neural Network, Recurrent Neural Network, e.g. a Long Short-Term Memory Network, or Attention based Networks. This allows for an easy and optimum adaption of the neurological condition detection algorithm to typical neurological condition onset pattern occurring on real patients.

The computer can be located in the cloud (server type computer) or be any commercially available computer, like a PC, Laptop, Notebook, Tablet or Smartphone, or a microprocessor, microcontroller, DSP or FPGA, or a combination of such elements. The computer program can be stored on a non-transitory computer-readable medium.

As far as a closed-loop control is mentioned, a closed-loop control differs from a open-loop control in that a closed-loop control has a feedback or feedback of measured or internal values, with which the generated output values of the closed-loop control are influenced in turn in the sense of a closed-loop control circuit. In a closed-loop control system, only a variable is controlled without such feedback or feedback.

Further exemplary embodiments of the disclosure are described using the following figures, which show in:
- Figure 1: a patient with an implanted neurostimulation device,
- Figure 2: details of the implanted neurostimulation device,
- Figure 3: example result of automatic electrode selection using a first approach,

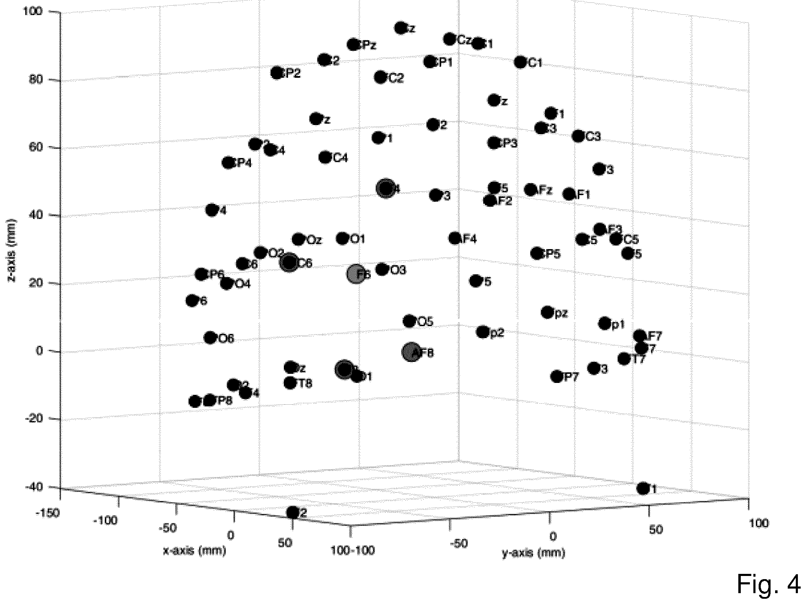

- Figure 4: example result of automatic electrode selection using a second approach.

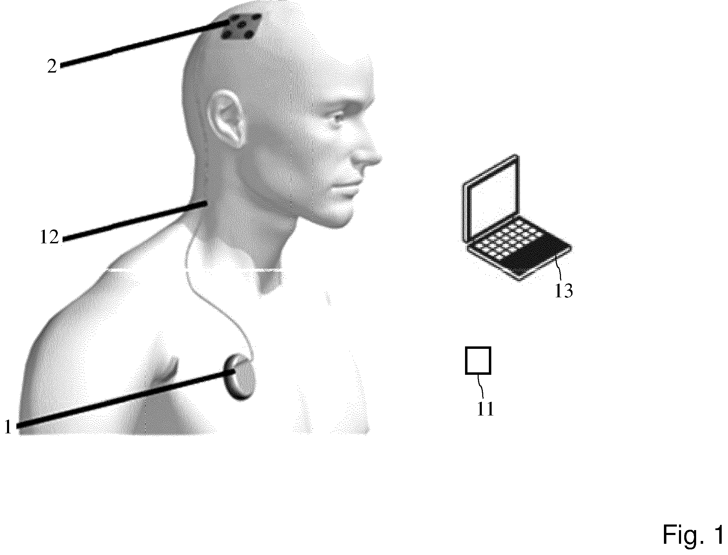

Figure 1 shown a neurostimulation system implanted in a patient. The neurostimulation system comprises an implantable neurostimulation device 1, which is connected via electrical leads 12 with a target electrode arrangement 2. The target electrode arrangement 2 may be placed on the outside of the skull of the patient, below the scalp of the patient.

Figure 1 further shows an external computer 13 and as another external device a patient controller 11. Both, the computer 13 and the patient controller 11 may wirelessly communicate with the implantable neurostimulation device 1. For example, the computer 13 can be used as a clinical system to be used by a doctor, for example for programming the implantable neurostimulation device 1. The patient controller 11 can be used by the patient for checking the status the implantable neurostimulation device 1 or for activating specific stimulation modes. The patient controller 11 can also function as a recorder for logging seizure events or other events which the patient enters or which are transmitted from the implantable neurostimulation device 1. The patient controller 11 can also function as an alarm system which provides haptical and/or optical and/or acoustic feedback to the patient in case of a neurological event. The computer 13 can also be used for running the training computer program of the invention, for training the neurological condition detection algorithm. The computer 13 may also be used for programming the trained neurological condition detection algorithm into the implantable neurostimulation device 1.

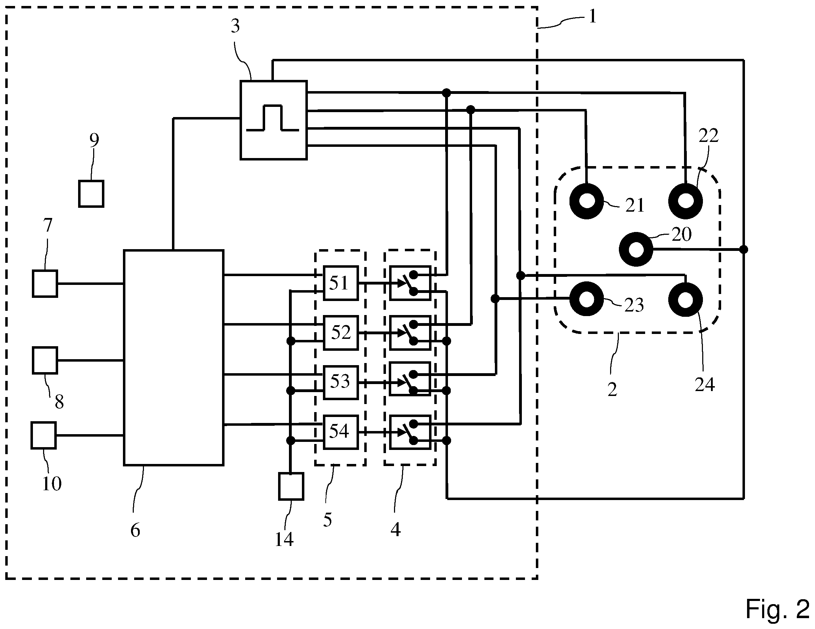

Figure 2 shows further details of the implantable neurostimulation device 1 and the target stimulation and/or recording electrode arrangement 2 in the representation of a block diagram similar to an electric circuit diagram. The neurostimulation device 1 comprises a control processor 6, a signal generation circuit 3, a charge balancing circuit 4, a protection circuit 5, sensors 7, 8, a battery pack 9 and a user input element 10. The neurostimulation device 1 is connected via the cables 12 to the electrode arrangement 2. As can be seen, the electrode arrangement 2 comprises a center electrode 20 and four stimulation electrodes 21, 22, 23, 24, which are located around the center electrode 20. The center electrode 20 can be a common ground electrode which means that the center electrode 20 is connected to the common ground of the neurostimulation device respective its neurostimulation device 1.

The control processor 6 can be a microcontroller unit (MCU) or any other unit, which can perform control steps via processing of computer programs, e.g. in the form of hardware, firmware or software programs.

The signal generation circuit 3 is able to create and deliver stimulation pulses to the stimulation electrodes 21, 22, 23, 24 upon command from the control processor 6. The signal generation circuit 3 may comprise amplifier components.

The control processor 6 can detect neuro-signals and/or brain activities through the sensors 7, 8. The detected neuro-signals and/or brain activities can be processed and used for event driven delivery of stimulation pulses to any of the stimulation electrodes 21, 22, 23, 24.

The battery pack 9 supplies the aforementioned elements of the power unit 1 with electrical energy. The battery pack 9 may comprise rechargeable batteries.

The control processor 6 is arranged for executing the trained neurological condition detection algorithm. The training of the neurological condition detection algorithm is done on the computer 13, as it will be described in the following.

The neurological condition detection algorithm for such a system must be trained on recordings configured similar to the subgaleal electrodes. Since there is a high similarity between EEG data from subcutaneous and proximate scalp electrodes in patients with neocortical epilepsy, we use surface EEG recordings obtained from electrode configurations representing the implanted subgaleal electrode for training and evaluation of the neurological condition detection algorithm. This approach could be used to pre-train an individualized detector using scalp-based neurological condition recordings prior to a device implantation.

Electrode selection can be performed to represent the target electrode arrangement 2 during long-term video-EEG monitoring of neurological conditions. Expert epileptologists may define the seizure onset zone (SOZ) by visual exploration of the seizure onset electrodes and considering inter-electrode distances. Our proposed methods automate the electrode selection by considering the geometrical dimensions of the system shown in Figure 1 and assure an optimal electrode selection that simulates the placement of the subgaleal recording electrodes. Next, a neurological condition detection algorithm is required that can perform well with a reduced number of electrodes with limited spatial coverage and has a low power consumption. This will allow the neurological condition detector to be integrated into a fully implantable intervention device. There are several publications about the development of neurological condition detection algorithms for either offline or online applications. However, the number of studies that consider the limitations for closed-loop applications is limited.

In our system, after development and implementation of two automatic electrode selection methods, we designed and implemented four energy-efficient neurological condition detection algorithms using Random Forest (RF) classifier, Support Vector Machine (SVM), Multi-Layer Perceptron (MLP), and Convolutional Neural Network (CNN) that can perform reliably with a reduced electrode set for detection of focal-onset seizures. Finally, we compared their detection performance to evaluate their suitability for implantable devices.

The EEG dataset used for training included surface EEG recordings of 50 patients with a total number of 358 seizures. Patients were selected, having the SOZ covered by electrodes positioned according to the 10-10 system in addition to the traditional 10-20 electrode layout. EEG data were recorded at a 250 Hz sampling rate on a 256-channels DC amplifier with a resolution of 24 bit. Electrodes were re-referenced to the centrally positioned electrode. For antialiasing, a low-pass filter with a cut-off frequency of 100 Hz was applied. EEG data from ten patients were used for training the hybrid model of the CNN. Therefore, to keep the test dataset consistent over all the classifiers, the remaining 40 patients with a total number of 286 seizures were used for evaluating the classifiers.

To improve the data quality and removing data contaminated with artifacts, first, data were filtered by a Chebyshev II band-pass (order:10, band stop= 40dB) with low and high cut-off frequencies of 0.1 Hz and 48 Hz. Next, very high amplitudes representing artifacts were removed from the analysis.

Electrode selection was performed to obtain an electrode set with the maximum number of electrodes covering the seizure onset area at inter-electrode distances below a threshold mapping to the design of the implantable system shown in Figure 1. To this end, for each seizure, a list of electrodes - considered to cover the SOZ(s) - was determined by expert epileptologists. Hereafter, based on this list, the minimum electrode set that includes all the seizure onset electrodes was determined. If the size of this electrode set was less than the number of implant electrodes (n=5), then, electrodes from the remaining seizure onset electrodes that were most frequently involved in the seizure onset were added to this electrode set. With this process, a list of five electrodes that are most likely to capture all the seizures was set for each patient.

### Method 1 (not part of the invention)

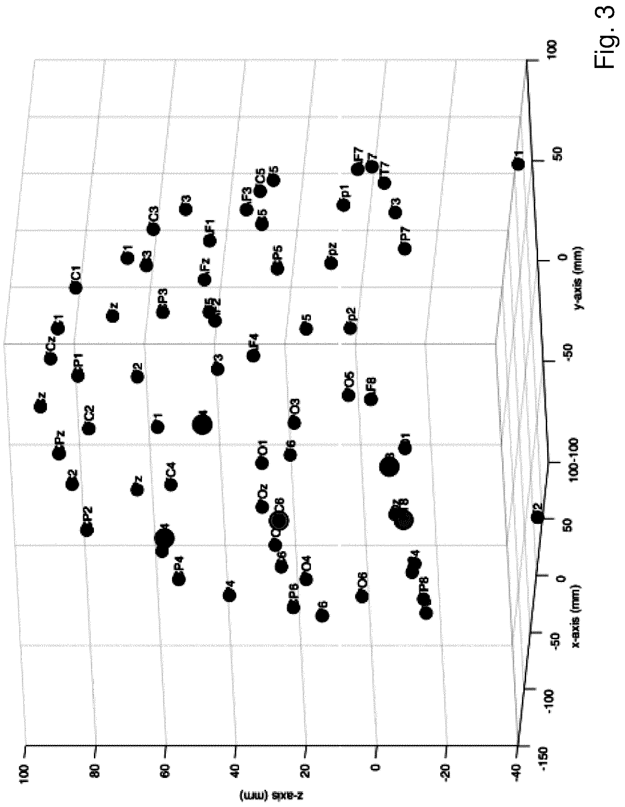

In this method, first, the mean of the five selected electrode coordinates was calculated and the nearest scalp electrode to this position was found. This electrode was considered as the center electrode. Any of the selected five electrodes that had a distance from the center electrode greater than the threshold distance was excluded from the list. As depicted in figure 3, this can be visualized as drawing a sphere of threshold radius with center at the selected center electrode and checking if other selected electrodes lie inside this sphere. Now, the shortlisted electrodes contained the center electrode and neighboring seizure onset electrodes. The remaining electrodes with minimum distance from the center electrode replaced the removed electrodes from the list of five electrodes.

### Method 2

In this method, in each step, one electrode was selected as the central electrode and similar to method 1, the number of electrodes from the initial SOZ electrodes whose distance from the central electrode was smaller than the threshold distance was counted. This process was repeated for all EEG electrode positions over the scalp, and a list of the selected initial SOZ electrodes enclosed with each electrode was generated. The electrode that contained the maximum number of initial SOZ electrodes was chosen as the optimal central electrode. If required, electrodes with a minimum distance from the central electrode were added to the list to yield exactly five electrodes for seizure detection.

Several features in the time and frequency domain were selected for seizure detection. Time-domain features were mean, maximum, mean absolute deviation, variance, skewness, kurtosis, line length, autocorrelation, and entropy. Frequency domain features included mean, maximum, and variance of the power spectrum, power in the theta (4-8 Hz), beta (13-30 Hz), and gamma band (30-45 Hz), spectral entropy, and epileptogenicity index. These features were used for classification by Random Forest, SVM, and MLP classifiers. For SVM and MLP, as the range of calculated features affect their weight as well as the subsequent decision boundaries, features were scaled for classification.

### 1) Random Forest

The number of binary decision trees was set to 100. We selected entropy for impurity measurement as the branching index. Four features were randomly selected at each node. To keep the size of the trees limited, the maximum depth of the trees was limited to 10. Bootstrap samples were used during building decision trees. The sample weights for each class were adjusted inversely proportional to class frequencies in the input data. The "leave one out" method was used for crossvalidation.

### 2) Support Vector Machine (SVM)

We selected the Gaussian Radial Basis Function (RBF) as the kernel function for handling non-linearity between the features and class labels. Two hyperparameters needed to be set for this purpose: the kernel coefficient of the Gaussian function was set to 0.01, and the penalty parameter of the error term, which behaves as a regularization parameter for the SVM, was set to 0.1. The sample weights for each class were adjusted so that they were inversely proportional to class frequencies in the input data. The "leave-one-out" method was used for cross-validation.

### 3) Multi-layer Perceptron (MLP)

The MLP network consists of at least three layers of nodes: an input layer, one or more hidden layers, and an output layer. We implemented an MLP classifier consisting of one hidden layer with 20 neurons. We selected "Adam" as the solver for weight optimization. The logistic sigmoid function was selected as the activation function and an adaptive learning rate was selected to schedule for weight updates. The L2 penalty (regularization term) parameter was set to 10⁻⁴. The "leave one out" method was used for cross-validation.

### 4) Convolutional Neural Network (CNN)

A CNN consists of an input layer, multiple hidden layers, and an output layer. The hidden layers consist of convolutional layers, pooling layers, and fully connected layers. The architecture of our proposed CNN is shown in Table I. In the first layer, to be able to learn spatio-temporal patterns efficiently, we used a kernel size that extends over all the channels and detection time window (2 seconds=500 data points). In all hidden layers, we used batch normalization after the convolutions and Rectified Linear Units (ReLu) were used as the activation function. Dropout regularization was applied during the training to reduce overfitting. In the two last layers, we used two fully connected layers.

The following table shows a preferred architecture of our proposed CNN:

| ***Layer*** | ***Operation*** | ***Output*** |
|---|---|---|
| | Input (4 × 500) | C × 500 × 1 |
| 1 | 15 × Conv2D (4 × 21) | 1 × 480 × 15 |
| 2 | MaxPool2D (1 × 4) | 1 × 120 × 15 |
| | Dropout (0.2) | 1 × 120 × 15 |
| 3 | 15 × Conv2D (1 × 9) | 1 × 112 × 15 |
| 4 | MaxPool2D (1 × 4) | 1 × 28 × 15 |
| | Dropout (0.2) | 1 × 28 × 15 |
| 5 | 10 × Conv2D (1 × 5) | 1 × 24 × 10 |
| 6 | MaxPool2D (1 × 2) | 1 × 12 × 10 |
| | Dropout (0.2) | 1 × 12 × 10 |
| 7 | 10 × Conv2D (1 × 5) | 1 × 8 × 10 |
| | Dropout (0.2) | 1 × 8 × 10 |
| 8 | Dense (8) | 8 |
| 9 | Dense (4) | 4 |
| 10 | Sigmoid | 1 |

For training, because the available data for each patient was limited, we used the transfer learning method that consists of freezing the bottom layers in a model and only training the top layers. Therefore, we pre-trained the network over data from 10 patients, and subsequently, for each of the remaining 40 patients, the last convolution layer and the two fully connected layers were fine-tuned using the patient-specific data. Because the classes are imbalanced, the class indices were weighted to balance the weighting of the loss function during the training. Each model was trained with a batch size of 512 for 500 epochs. For weight optimization, we used Adam solver with a learning rate of 10⁻³. We used binary cross-entropy as the loss function. For evaluation, we used 3-fold cross-validation.

## Claims

1. A computer program for training a neurological condition detection algorithm to be used for neurological condition detection in an implantable neurostimulation device having a target electrode arrangement having five electrodes which are arranged in a pseudo-Laplacian pattern having a center electrode and four stimulation electrodes which surround the center electrode, the computer program configured to execute the following steps:
a) receiving EEG data in a computer which executes the computer program, the EEG data being recorded by at least one EEG from at least one patient using a 10-20 or 10-10 or any other high density EEG electrode system,
b) identifying neurological activity in the EEG data, which corresponds to a neurological condition, based upon neurological condition identification tags comprising information identifying the time and location of a neurological condition included in the EEG data and/or input in the computer,
c) selecting a subset of five electrode channels out of the available electrode channels in the EEG data c22) one electrode is selected as the central electrode and the number of electrodes from an initial seizure onset zone electrodes whose distance from the central electrode is smaller than a threshold distance is counted;
this process is repeated for all EEG electrode positions over the scalp, and a list of selected initial seizure onset zone electrodes enclosed with each electrode is generated; the electrode that contained the maximum number of initial seizure onset zone electrodes is chosen as the central electrode; c23) remaining electrodes with a minimum distance from the central electrode are added to the list to yield exactly five electrodes for seizure detection,
d) training a neurological condition detection algorithm by using the EEG data only of the selected subset of electrode channels.

2. The computer program of any of the preceding claims, wherein step d) comprises the steps:
d1) calculating linear combinations of the EEG data of the selected subset of electrode channels, e.g. calculating linear combinations representing bipolar or quadrupolar electrode channels,
d2) training the neurological condition detection algorithm by using calculated linear combinations of the EEG data.

3. The computer program of any the preceding claims, wherein the neurological condition detection algorithm is an artificial intelligence algorithm, e.g. Random Forest, Support Vector Machine, Multi-layer Perceptron, Convolutional Neural Network, Long Short-Term Memory Network.

4. The computer program of any of the preceding claims, wherein the computer program comprises at least two training cycles of the neurological condition detection algorithm:
e) in a first training cycle a general training of the neurological condition detection algorithm is done using the EEG data of one or more patients,
f) in a second training cycle a patient specific training of the neurological condition detection algorithm is done using the EEG data only of the patient to which the neurological condition detection algorithm shall be applied and/or using the EEG data of another patient having similar neurological condition onset pattern as the patient to which the neurological condition detection algorithm shall be applied.

5. The computer program of any of the preceding claims, wherein the computer program is arranged for evaluating data tags which are assigned to the EEG data which are input in the computer which executes the computer program, wherein the data tags are used for selecting the subset of electrode channels out of the available electrode channels.

6. A method of programming an implantable neurostimulation device, comprising the following steps:
g) running a computer program of any the preceding claims on a computer,
h) programming the neurological condition detection algorithm trained by the computer program into the implantable neurostimulation device.

7. The method of claim 6, wherein in step g) the computer program is run on an external computer which is not part of the implantable neurostimulation device.

8. An implantable neurostimulation device comprising a computer program in the form of a neurological condition detection algorithm for detecting neurological conditions from EEG data which has been trained and/or is being trained by a computer program according to any of claims 1 to 5.

9. The implantable neurostimulation device of claim 8, wherein the neurological condition detection algorithm for detecting neurological conditions is an artificial intelligence algorithm, e.g. Random Forest, Support Vector Machine, Multi-layer Perceptron, Convolutional Neural Network, Long Short-Term Memory Network.

10. The implantable neurostimulation device of claim 8, wherein the implantable neurostimulation device is a closed-loop neurostimulator which is arranged for recording EEG signals, for calculating stimulation signals based upon the recorded EEG signals and for outputting the stimulation signals.

## Patentansprüche

1. Ein Computerprogramm zum Trainieren eines Algorithmus zur Detektion eines neurologischen Zustands, der zur Detektion eines neurologischen Zustands in einer implantierbaren Neurostimulationsvorrichtung verwendet werden soll, die eine Zielelektrodenanordnung mit fünf Elektroden aufweist, welche in einem Pseudo-Laplace-Muster mit einer Mittelelektrode und vier die Mittelelektrode umgebenden Stimulationselektroden angeordnet sind, wobei das Computerprogramm so konfiguriert ist, dass es die folgenden Schritte ausführt:
a) Empfangen von EEG-Daten in einem Computer, der das Computerprogramm ausführt, wobei die EEG-Daten von mindestens einem EEG von mindestens einem Patienten unter Verwendung eines 10-20 oder 10-10 oder eines anderen hochdichten EEG-Elektrodensystems aufgezeichnet werden,
b) Identifizieren neurologischer Aktivität in den EEG-Daten, die einem neurologischen Zustand entspricht, basierend auf Identifikations-Tags für neurologische Zustände, die Informationen zur Identifizierung der Zeit und des Ortes eines neurologischen Zustands enthalten, die in den EEG-Daten enthalten und/oder in den Computer eingegeben sind,
c) Auswählen einer Teilmenge von fünf Elektrodenkanälen aus den verfügbaren Elektrodenkanälen in den EEG-Daten, wobei c22) eine Elektrode als die zentrale Elektrode ausgewählt wird und die Anzahl der Elektroden aus Elektroden einer initialen Anfallsursprungszone, deren Abstand von der zentralen Elektrode kleiner als ein Schwellenabstand ist, gezählt wird; dieser Prozess wird für alle EEG-Elektrodenpositionen über der Kopfhaut wiederholt, und eine Liste von ausgewählten Elektroden der initialen Anfallsursprungszone, die von jeder Elektrode umschlossen sind, wird erstellt; die Elektrode, die die maximale Anzahl von Elektroden der initialen Anfallsursprungszone enthielt, wird als die zentrale Elektrode gewählt;
c23) verbleibende Elektroden mit einem minimalen Abstand von der zentralen Elektrode werden der Liste hinzugefügt, um genau fünf Elektroden für die Anfallsdetektion zu ergeben,
d) Trainieren eines Algorithmus zur Detektion eines neurologischen Zustands unter Verwendung nur der EEG-Daten der ausgewählten Teilmenge von Elektrodenkanälen.

2. Das Computerprogramm nach einem der vorhergehenden Ansprüche, wobei Schritt d) die folgenden Schritte umfasst:
d1) Berechnen von Linearkombinationen der EEG-Daten der ausgewählten Teilmenge von Elektrodenkanälen, z.B. Berechnen von Linearkombinationen, die bipolare oder quadrupolare Elektrodenkanäle repräsentieren,
d2) Trainieren des Algorithmus zur Detektion eines neurologischen Zustands unter Verwendung der berechneten Linearkombinationen der EEG-Daten.

3. Das Computerprogramm nach einem der vorhergehenden Ansprüche, wobei der Algorithmus zur Detektion eines neurologischen Zustands ein Algorithmus der künstlichen Intelligenz ist, z.B. Random Forest, Support Vector Machine, Multi-Layer Perceptron, Convolutional Neural Network, Long Short-Term Memory Network.

4. Das Computerprogramm nach einem der vorhergehenden Ansprüche, wobei das Computerprogramm mindestens zwei Trainingszyklen des Algorithmus zur Detektion eines neurologischen Zustands umfasst:
e) in einem ersten Trainingszyklus wird ein allgemeines Training des Algorithmus zur Detektion eines neurologischen Zustands unter Verwendung der EEG-Daten von einem oder mehreren Patienten durchgeführt,
f) in einem zweiten Trainingszyklus wird ein patientenspezifisches Training des Algorithmus zur Detektion eines neurologischen Zustands nur unter Verwendung der EEG-Daten des Patienten durchgeführt, auf den der Algorithmus zur Detektion eines neurologischen Zustands angewendet werden soll, und/oder unter Verwendung der EEG-Daten eines anderen Patienten, der ein ähnliches Anfallsursprungsmuster wie der Patient hat, auf den der Algorithmus zur Detektion eines neurologischen Zustands angewendet werden soll.

5. Das Computerprogramm nach einem der vorhergehenden Ansprüche, wobei das Computerprogramm so eingerichtet ist, dass es Daten-Tags auswertet, die den EEG-Daten zugewiesen sind, die in den Computer eingegeben werden, der das Computerprogramm ausführt, wobei die Daten-Tags zur Auswahl der Teilmenge von Elektrodenkanälen aus den verfügbaren Elektrodenkanälen verwendet werden.

6. Ein Verfahren zum Programmieren einer implantierbaren Neurostimulationsvorrichtung, umfassend die folgenden Schritte:
g) Ausführen eines Computerprogramms nach einem der vorhergehenden Ansprüche auf einem Computer,
h) Programmieren des durch das Computerprogramm trainierten Algorithmus zur Detektion eines neurologischen Zustands in die implantierbare Neurostimulationsvorrichtung.

7. Das Verfahren nach Anspruch 6, wobei in Schritt g) das Computerprogramm auf einem externen Computer ausgeführt wird, der nicht Teil der implantierbaren Neurostimulationsvorrichtung ist.

8. Eine implantierbare Neurostimulationsvorrichtung, umfassend ein Computerprogramm in Form eines Algorithmus zur Detektion eines neurologischen Zustands zum Detektieren neurologischer Zustände aus EEG-Daten, der durch ein Computerprogramm nach einem der Ansprüche 1 bis 5 trainiert wurde und/oder trainiert wird.

9. Die implantierbare Neurostimulationsvorrichtung nach Anspruch 8, wobei der Algorithmus zur Detektion eines neurologischen Zustands zum Detektieren neurologischer Zustände ein Algorithmus der künstlichen Intelligenz ist, z.B. Random Forest, Support Vector Machine, Multi-Layer Perceptron, Convolutional Neural Network, Long Short-Term Memory Network.

10. Die implantierbare Neurostimulationsvorrichtung nach Anspruch 8, wobei die implantierbare Neurostimulationsvorrichtung ein geschlossener Neurostimulator ist, der zum Aufzeichnen von EEG-Signalen, zum Berechnen von Stimulationssignalen basierend auf den aufgezeichneten EEG-Signalen und zum Ausgeben der Stimulationssignale eingerichtet ist.

## Revendications

1. Un programme d'ordinateur pour l'entraînement d'un algorithme de détection de condition neurologique destiné à être utilisé pour la détection de condition neurologique dans un dispositif de neurostimulation implantable ayant un agencement d'électrodes cible comportant cinq électrodes qui sont agencées selon un motif pseudo-laplacien ayant une électrode centrale et quatre électrodes de stimulation qui entourent l'électrode centrale, le programme d'ordinateur étant configuré pour exécuter les étapes suivantes :
a) recevoir des données EEG dans un ordinateur qui exécute le programme d'ordinateur, les données EEG étant enregistrées par au moins un EEG d'au moins un patient en utilisant un système d'électrodes EEG 10-20 ou 10-10 ou tout autre système d'électrodes EEG à haute densité,
b) identifier une activité neurologique dans les données EEG, qui correspond à une condition neurologique, sur la base d'étiquettes d'identification de condition neurologique comprenant des informations identifiant le moment et l'emplacement d'une condition neurologique incluses dans les données EEG et/ou entrées dans l'ordinateur,
c) sélectionner un sous-ensemble de cinq canaux d'électrodes parmi les canaux d'électrodes disponibles dans les données EEG, dans lequel c22) une électrode est sélectionnée comme électrode centrale et le nombre d'électrodes d'une zone de début de crise initiale dont la distance par rapport à l'électrode centrale est inférieure à une distance seuil est compté ; ce processus est répété pour toutes les positions d'électrodes EEG sur le scalp, et une liste d'électrodes de la zone de début de crise initiale sélectionnées incluses avec chaque électrode est générée ; l'électrode qui contenait le nombre maximum d'électrodes de la zone de début de crise initiale est choisie comme électrode centrale ;
c23) les électrodes restantes ayant une distance minimale par rapport à l'électrode centrale sont ajoutées à la liste pour obtenir exactement cinq électrodes pour la détection de crise,
d) entraîner un algorithme de détection de condition neurologique en utilisant uniquement les données EEG du sous-ensemble de canaux d'électrodes sélectionné.

2. Le programme d'ordinateur de l'une quelconque des revendications précédentes, dans lequel l'étape d) comprend les étapes suivantes :
d1) calculer des combinaisons linéaires des données EEG du sous-ensemble de canaux d'électrodes sélectionné, par ex. calculer des combinaisons linéaires représentant des canaux d'électrodes bipolaires ou quadripolaires,
d2) entraîner l'algorithme de détection de condition neurologique en utilisant les combinaisons linéaires calculées des données EEG.

3. Le programme d'ordinateur de l'une quelconque des revendications précédentes, dans lequel l'algorithme de détection de condition neurologique est un algorithme d'intelligence artificielle, par ex. Forêt Aléatoire, Machine à Vecteurs de Support, Perceptron Multi-couches, Réseau Neuronal Convolutif, Réseau de Mémoire à Court et Long Terme.

4. Le programme d'ordinateur de l'une quelconque des revendications précédentes, dans lequel le programme d'ordinateur comprend au moins deux cycles d'entraînement de l'algorithme de détection de condition neurologique :
e) dans un premier cycle d'entraînement, un entraînement général de l'algorithme de détection de condition neurologique est effectué en utilisant les données EEG d'un ou plusieurs patients,
f) dans un second cycle d'entraînement, un entraînement spécifique au patient de l'algorithme de détection de condition neurologique est effectué en utilisant uniquement les données EEG du patient auquel l'algorithme de détection de condition neurologique doit être appliqué et/ou en utilisant les données EEG d'un autre patient ayant un schéma de début de condition neurologique similaire à celui du patient auquel l'algorithme de détection de condition neurologique doit être appliqué.

5. Le programme d'ordinateur de l'une quelconque des revendications précédentes, dans lequel le programme d'ordinateur est agencé pour évaluer des étiquettes de données qui sont assignées aux données EEG qui sont entrées dans l'ordinateur qui exécute le programme d'ordinateur, dans lequel les étiquettes de données sont utilisées pour sélectionner le sous-ensemble de canaux d'électrodes parmi les canaux d'électrodes disponibles.

6. Un procédé de programmation d'un dispositif de neurostimulation implantable, comprenant les étapes suivantes :
g) exécuter un programme d'ordinateur de l'une quelconque des revendications précédentes sur un ordinateur,
h) programmer l'algorithme de détection de condition neurologique entraîné par le programme d'ordinateur dans le dispositif de neurostimulation implantable.

7. Le procédé de la revendication 6, dans lequel à l'étape g) le programme d'ordinateur est exécuté sur un ordinateur externe qui ne fait pas partie du dispositif de neurostimulation implantable.

8. Un dispositif de neurostimulation implantable comprenant un programme d'ordinateur sous la forme d'un algorithme de détection de condition neurologique pour détecter des conditions neurologiques à partir de données EEG qui a été entraîné et/ou est en cours d'entraînement par un programme d'ordinateur selon l'une quelconque des revendications 1 à 5.

9. Le dispositif de neurostimulation implantable de la revendication 8, dans lequel l'algorithme de détection de condition neurologique pour détecter des conditions neurologiques est un algorithme d'intelligence artificielle, par ex. Forêt Aléatoire, Machine à Vecteurs de Support, Perceptron Multi-couches, Réseau Neuronal Convolutif, Réseau de Mémoire à Court et Long Terme.

10. Le dispositif de neurostimulation implantable de la revendication 8, dans lequel le dispositif de neurostimulation implantable est un neurostimulateur en boucle fermée qui est agencé pour enregistrer des signaux EEG, pour calculer des signaux de stimulation sur la base des signaux EEG enregistrés et pour émettre les signaux de stimulation.
